# EUROPEAN PATENT SPECIFICATION

(11) **EP 2 205 541 B1**
(45) Date of publication and mention of the grant of the patent: **27.11.2013**
(21) Application number: 08802835.2
(22) Date of filing: 07.10.2008
(51) Int. Cl.: C07C 5/333, C07C 7/04, C07C 15/46, C07C 2/66

(54) **INTEGRATED PROCESS FOR THE PRODUCTION OF VINYL AROMATIC HYDROCARBONS**
INTEGRIERTER PROZESS ZUR HERSTELLUNG VON VINYLAROMATISCHEN VERBINDUNGEN
PROCEDE INTEGRE DE PRODUCTION D'HYDROCARBURES VINYLES AROMATIQUES

(30) Priority: 09.10.2007 IT MI20071952
(43) Date of publication of application: 14.07.2010
(73) Proprietor: versalis S.p.A., 20097 San Donato Milanese (MI) (IT)
(72) Inventor: BENCINI, Elena, 46030 Virgilio (IT); DEL SEPPIA, Alessandro, 46047 Porto Mantovano (IT); GALEOTTI, Armando, 46023 Gonzaga (IT)
(74) Representative: Maiwald Patentanwalts GmbH
(86) International application number: PCT/EP2008/008510
(87) International publication number: WO 2009/046977

(56) References cited:
- WO-A-2005/063659
- WO-A-2007/073918

## Description

The present invention relates to an integrated process for the production of vinyl aromatic hydrocarbons.

More specifically, the present invention relates to a process for the production of styrene, integrated with a synthesis section of ethyl benzene.

As is known, integrated processes for the production of vinyl aromatic hydrocarbons (WO 2005/063659), for example styrene, comprise the preparation of an intermediate alkyl aromatic product, for example ethyl benzene, and the subsequent dehydrogenation of the latter to give the desired product. This operational cycle implies a series of unitary operations which give rise to a series of reaction by-products which must be suitably managed to improve the economical aspect of the overall process. Among the by-products which are formed in this production cycle, the aromatic hydrocarbon (benzene) is particularly important, which, as it is one of the starting raw materials of the whole integrated cycle, can be recovered and recycled to the production system.

The present invention therefore relates to an integrated process for the production of vinyl aromatic hydrocarbons characterized by an improved procedure for the recycling of the aromatic hydrocarbon component, in particular that coming from the dehydrogenation section of alkyl aromatic hydrocarbons. This procedure comprises, among other things, the feeding of the recycled aromatic hydrocarbon to an alkylation/transalkylation guard unit in order to simply and effectively increase the life cycle of the zeolite-based catalyst of the main alkylation and transalkylation reactors.

In particular, the present invention relates to an integrated process for the production of styrene or α-methyl styrene characterized by an improved procedure for the recycling of benzene, coming from the dehydrogenation section of ethyl benzene and isopropyl benzene, which, after being treated in purification sections, is fed together with fresh feeding benzene, together with the benzene recycled from the separation section of the alkylated mixture, to an alkylation/transalkylation guard unit which guarantees its quality before being sent to the main alkylation and transalkylation reactors.

The synthesis of alkyl aromatic compounds is known in literature. European patent 432.814, for example, describes the synthesis of alkyl aromatic compounds, such as ethyl benzene or isopropyl benzene (cumene) by the reaction between an aromatic hydrocarbon, such as benzene, and an olefin, such as ethylene or propylene, in the presence of a Beta Zeolite, possibly modified by substitution of the aluminum with boron, gallium or iron.

More specifically, according to this patent, the reagents of the alkylation reaction are put in contact with the catalyst at a temperature ranging from 100 to 300°C, at a pressure ranging from 10 to 50 atm and with a flow-rate of the reagents which is such as to give a WHSV (Weight Hourly Space Velocity) ranging from 0.1 to 200 h⁻¹. Under these operating conditions, the catalyst shows a low life span, intended as the time lapse between two regenerations. This life duration of the catalyst, however, can be improved.

It is known, in fact, that one of the main reasons for a rapid deterioration in the activity of zeolitic-based catalysts lies in the poisoning effect caused by impurities contained in the reagents, in particular contained in the aromatic hydrocarbon, for example benzene.

On the basis of what is specified above, processes have been developed which allow the benzene to be pretreated before feeding it to the alkylation reaction. European patent 780.354, for example, describes a method for eliminating the oxygen and other impurities dissolved in the benzene which comprises stripping the oxygen with an inert gas and subsequently percolating the hydrocarbon through an alumina bed modified with silver.

International patent application WO 2005/61420 describes a process and apparatus for eliminating nitrogen-based compounds contained in a substrate for alkylation such as benzene, through the absorption of these nitrogenated compounds on molecular sieves.

In the known processes, however, and in others described in literature such as, for example, in US patent application 2006/194991 or in international patent application WO 98/07673, an inlet source for poisons for the alkylation catalyst also consists of the recycled streams.

It is in fact known that alkyl aromatic hydrocarbons such as ethyl benzene or cumene (isopropyl benzene) can be dehydrogenated to produce styrene and α-methylstyrene, in turn raw materials for a wide range of thermoplastic and/or thermosetting resins. At the end of the dehydrogenation phase, the styrene and α-methylstyrene are separated from the reaction mixture which comprises, among the by-products, a significant quantity of benzene which, for economical reasons, is preferably recovered to recycle it, when possible, to the alkylation section.

The recycled benzene, however, entrains significant quantities of impurities with it, for example higher olefins or nitrogenated compounds, which are difficult to separate and which are poisonous for the zeolitic alkylation catalysts. At times, the purification of the recycled benzene coming from the separation section of a dehydrogenation unit is so onerous that it is more convenient to recycle it to a plant for the production of aromatic compounds, if possible, or otherwise send it to thermo-destruction rather than purifying it.

The treatment for the removal of nitrogenated compounds by absorption, for example, requires a high substitution frequency of the adsorbing material which is generally too onerous for industrial applications. Furthermore, the difficulty involved in the continuous and on-line analytical monitoring of the efficacy of the removal of these nitrogenated compounds often causes the irreversible poisoning of the zeolitic catalyst in the alkylation and transalkylation production sections.

The Applicant has now found a process for the purification of the recycled aromatic hydrocarbon, coming from the dehydrogenation section of alkyl aromatic hydrocarbons, in an integrated system for the production of vinyl aromatic monomers, which is simple and extremely effective in neutralizing the impurities which accompany this product.

An object of the present invention therefore relates to an integrated process for the production of vinyl aromatic hydrocarbons, which comprises:
a) feeding a hydrocarbon stream comprising a fresh and recycled aromatic hydrocarbon to an alkylation section, comprising a transalkylation unit, together with a stream essentially consisting of a C₂-C₃ olefin;
b) feeding the reaction product coming from the alkylation section to a first separation section;
c) discharging from the first separation section a first stream consisting of a non-reacted aromatic hydrocarbon which is recycled, a second stream essentially consisting of a monoalkylated aromatic hydrocarbon, a third stream essentially consisting of dialkylated aromatic hydrocarbons and a fourth stream essentially consisting of a mixture of polyalkylated aromatic hydrocarbons;
d) feeding the second stream essentially consisting of the monoalkylated aromatic hydrocarbon of step (c) to a dehydrogenation section to produce a vinyl aromatic hydrocarbon and reaction by-products comprising the aromatic hydrocarbon;
e) feeding the reaction product coming from the dehydrogenation step (d) to a second separation section, comprising a distillation column of the aromatic hydrocarbon;
f) recovering from said second separation section a stream consisting of the vinyl aromatic hydrocarbon, with a purity higher than 99.7% by weight, and a stream comprising the aromatic hydrocarbon;
g) recycling the aromatic hydrocarbon coming from the first and second separation section to the alkylation section comprising the transalkylation unit;
   characterized by:
h) feeding a stream of polar solvent to the head of the distillation column of the aromatic hydrocarbon of step (e);
i) percolating the stream comprising the aromatic hydrocarbon coming from step (h) through at least one fixed bed consisting of molecular sieves or acid earth;
j) preparing a single guard reactor divided into two parts, the first containing a first catalytic bed, for an alkylation reaction, and the second containing a second catalytic bed, for a transalkylation reaction;
k) feeding to said first alkylation catalytic bed of said guard reactor, a first part (A1) of an overall aromatic stream comprising fresh feeding aromatic hydrocarbon, the recycled aromatic hydrocarbon coming from the first separation section (c) and from 0 to 100% by weight, preferably from 0 to 50% by weight referred to overall aromatic stream, of the recycled aromatic hydrocarbon stream coming from the purification phase (h)-(i) together with a stream essentially consisting of a C₂-C₃ olefin, with Al/olefin molar ratios ranging from 10/1 to 100/1;
l) feeding to said second transalkylation catalytic bed of said guard reactor the second part (A2) of the overall aromatic stream comprising fresh feeding aromatic hydrocarbon, the recycled aromatic hydrocarbon coming from the first separation section (c) and from 0 to 100% by weight, preferably from 50 to 100% by weight, of the recycled aromatic hydrocarbon stream coming from the purification phase (h) - (i) together with the third stream, essentially consisting of dialkylated aromatic hydrocarbons, of step (c), with A2/olefin molar ratios, calculated with respect to the total moles of olefin groups present as substituents in the dialkylated aromatic hydrocarbons, ranging from 2/1 to 18/1, preferably from 2.5/1 to 10/1; and
m) feeding the streams respectively leaving said first alkylation catalytic bed and said second transalkylation catalytic bed, of said single guard reactor, to the alkylation section comprising the transalkylation unit.

According to the present invention, the aromatic hydrocarbon fed to the alkylation section can be selected from those having from 6 to 9 carbon atoms but is preferably benzene. Another aromatic hydrocarbon which can be used in the process, object of the present invention, is, for example, toluene.

The preferred aromatic hydrocarbon is benzene refinery grade with a purity higher than or equal to 95% by weight.

The C₂-C₃ olefin stream, for example ethylene or propylene, also refinery grade with a purity higher than or equal to 95% by weight, is fed to the alkylation section together with the fresh and recycled aromatic hydrocarbon. The two streams, aromatic and olefin, are fed to the alkylation unit so as to have aromatic/olefin molar ratios which are such as to satisfy current technology requirements, generally from 2 to 50, preferably from 3 to 10.

The alkylation reaction is carried out with conventional systems, for example it can be carried out according to the method described in European patent 432.814.

Any alkylation reactor can be used in the process, object of the present invention. Fixed bed or fluid bed reactors, carrier bed reactors, reactors operating with a slurry mixture and catalytic distillation reactors, can be used, for example.

The preferred alkylation catalysts can be selected from synthetic and natural porous crystalline solids based on silicon and aluminum, such as acid zeolites in which the silicon/aluminum atomic ratio ranges from 5/1 to 200/1. In particular, Y, beta, omega zeolites, mordenite and porous crystalline solids MCM-22, MCM-36, MCM-49 are preferred. Alternatively, it is possible to use synthetic zeolites of the ZSM group in which the silicon/aluminum atomic ratio ranges from 20/1 to 200/1, such as ZSM-5 zeolite.

The alkylation reaction can be effected under temperature and pressure conditions which depend, in addition to the catalyst selected, also on the type of reactor and reagents selected. In the case of the alkylation of benzene with ethylene, the reaction temperature generally ranges from 50 to 450°C. More specifically, with zeolitic catalysts, for processes in gas phase, with both fixed and mobile beds, the temperature preferably ranges from 300 to 450°C or from 180 to 250°C for processes in liquid phase, whereas in the case of catalytic distillation reactors, operating in mixed gas-liquid phase, the reaction temperature, variable along the whole catalytic bed, ranges from 140 to 350°C, preferably from 200 to 300°C. The pressure inside the alkylation reactor is kept at values ranging from 0.3 to 6 MPa, preferably from 0.4 to 5 MPa.

The aromatic stream leaving the alkylation reactor is treated with conventional distillation means, for example plate columns, to recover the reaction product from non-converted reagents and from the reaction by-products. In particular, the separation system substantially and preferably consists of a series of at least three distillation columns from the first of which the non-reacted aromatic compound is recovered and recycled. From the second distillation column, the monoalkyl-substituted aromatic compound, for example ethyl benzene, is recovered and fed to the dehydrogenation section, whereas from the third distillation column the dialkylated aromatic compounds are recovered from the head and sent to a transalkylation unit, and the heavy products, essentially consisting of polyalkylated products, tetralins and alkyl-substituted diphenyl ethanes can be recovered from the bottom and sent for thermal-destruction or fed to the second separation section as polymerization retardant additives of the dehydrogenated compound and/or as diluents of the column-bottom products.

The mono-alkylated aromatic product is fed to the catalytic dehydrogenation section, which comprises one or more reactors operating with a fixed bed or fluid bed, preferably with a fixed bed, whereas the dialkylated product is sent to a conventional transalkylation unit present in the alkylation section.

The transalkylation reaction also takes place according to the conventional systems. It is possible to operate, for example, in liquid phase with a zeolitic catalyst of the Y, beta zeolite type, or mordenite, preferably Y or beta zeolite. The transalkylation reaction can be carried out according to what is described in European patent 847.802.

In the case of the transalkylation of diethyl benzene with benzene, the benzene/ethylene molar ratio, calculated with respect to the total moles of ethylene present as substituent in the diethyl benzenes, ranges from 2/1 to 18/1, preferably from 2.5/1 to 10/1. The temperature in the reactor is maintained at 50 to 350°C, preferably from 130 to 290°C, whereas the pressure is maintained at 0.02 to 6 MPa, preferably from 0.4 to 5 MPa.

The dehydrogenation reaction with a fixed bed reactor of the mono-alkylated aromatic product takes place at a temperature ranging from 500 to 700°C, preferably from 550 to 650°C, at a pressure ranging from 0.02 to 0.15 MPa, in the presence of a catalyst based on iron oxide and potassium carbonate containing other metallic compounds in small quantities acting as promoters.

When the vinyl aromatic monomer produced in the dehydrogenation section is styrene, the dehydrogenation can be effected, for example, with a fixed bed catalyst by feeding a mixture of ethyl benzene vapour and water vapour, in a water/ethyl benzene molar ratio ranging from 5/1 to 15/1, preferably from 6/1 to 12/1, on a first reactor in which a partial conversion of the ethyl benzene takes place. The reacted mixture leaving the first reactor is fed to a second reactor, after the temperature has been brought back to the required value by means of a heat exchanger. The reaction mixture in which the ethyl benzene has been converted for at least 60%, is cooled and condensed before being sent to the separation section. If required, at the outlet of the second reactor, it is possible to provide a third reactor for increasing the conversion of ethyl benzene up to and over 70%.

The reaction product leaving the dehydrogenation section is fed to a second separation section for the recovery of the dehydrogenated product, or vinyl aromatic monomer, for example styrene. In general, the separation section for the recovery of the dehydrogenated product can be effected according to what is described in international patent application WO 2005/63659.

The second separation section comprises a series of distillation columns, for example four or five plate distillation columns. The vinyl aromatic hydrocarbon (styrene) is recovered from this section with a purity degree higher than 99.7% by weight, for example higher than 99.9% by weight, and said aromatic hydrocarbon from the head of one of these columns, the so-called aromatic hydrocarbon column.

The aromatic hydrocarbon, for example benzene formed as by-product of the dehydrogenation reaction, can be recycled. In the case of the dehydrogenation of ethyl benzene to styrene, benzene containing olefins/C₅-C₇ dienes, linear or cyclic, is formed, together with nitrogenated compounds deriving from the degradation of additives added in the production cycle as polymerization inhibitors. If the aromatic hydrocarbon is recycled to the alkylation/transalkylation section, these impurities must be eliminated as they represent a poison for the respective catalysts.

According to the present invention, the purification of the aromatic hydrocarbon recovered from the second distillation section can be effected by feeding a stream of polar solvent, for example demineralized water, to the head or into the reflux accumulator of the aromatic hydrocarbon column, under the temperature and pressure conditions present in the column head. A stream in vapour phase essentially consisting of solvent and aromatic hydrocarbon is recovered from the head of said column, and, after condensation, is separated into two phases, one consisting of the aromatic hydrocarbon, without nitrogenated substances or with a reduced content of them, and the other of the solvent (water) containing said nitrogenated substances.

According to a preferred embodiment of the present invention, the aromatic hydrocarbon thus obtained can undergo a second treatment/washing with the polar solvent, for example in a section comprising a washing tower, with plates or filling, or a rain tower, where the solvent in liquid phase descends downwards and the aromatic hydrocarbon in vapour phase rises towards the top of the tower, from whose head it is then recovered and condensed. Alternatively, the second treatment/washing with the polar solvent can be effected by simply mixing the two liquids in a container and then separating the two phases through a demixer.

At the end of the washing phase with a solvent, the aromatic hydrocarbon is fed to a second treatment section comprising one or more fixed beds with molecular sieves or acid earth through which the aromatic hydrocarbon is percolated. The percolation phase takes place at temperatures ranging from 25 to 300°C, preferably from 30 to 250°C, more preferably from 35 to 220°C, at a pressure which is such as to maintain the aromatic hydrocarbon in liquid state. The percolation is effected on beds with molecular sieves or acid earth selected from amorphous silico-aluminates with a silicon/aluminum ratio lower than 100, generally ranging from 25 to 90. An example of a molecular sieve or acid earth with the above characteristics is montmorillonites.

According to a preferred embodiment of the present invention, after the aromatic hydrocarbon has been subjected to treatment with molecular sieves or acid earth, it can be subjected to a selective hydrogenation treatment. This treatment can be carried out in a specific reactor operating at a pressure ranging from 0.5 to 2 MPa, at a temperature ranging from 80 to 150°C and in the presence of a catalyst consisting of palladium supported on alumina.

At the end of the above treatments, the aromatic hydrocarbon by-product of the dehydrogenation section can be recycled to the alkylation section, comprising the transalkylation unit, together with the aromatic hydrocarbon coming from the first separation section and fresh feeding hydrocarbon. Thanks to the recycling mode described above, which allows the entrained impurities to be substantially definitively eliminated, it is possible to considerably increase the life cycle of the zeolite-based catalysts used for the alkylation and transalkylation reactions.

In total, the overall aromatic stream essentially consists of 8-20% by weight of fresh aromatic hydrocarbon, 79.8-90% by weight of recycled aromatic hydrocarbon coming from the first separation section and 0.2-2% by weight of purified aromatic hydrocarbon coming from the second separation section.

Furthermore, according to the present invention, the overall aromatic hydrocarbon stream, consisting of recycled aromatic hydrocarbon of the first and second separation section and the fresh feeding hydrocarbon, is fed to a single reactor, acting as guard, in which the two catalytic beds are arranged separated, for an alkylation reaction and a transalkylation reaction respectively. The reaction conditions inside the guard reactor, in the two separate reaction areas, are substantially identical to those present in the two main alkylation and transalkylation reaction units, with the exception of the molar ratios between the re-agents.

In particular, a fraction A1, equal to 60-80% of the overall aromatic stream, is fed to the section of the guard reactor containing the alkylation catalytic bed, together with a fraction of the total C₂-C₃ olefin stream with A1/olefin molar ratios ranging from 10/1 to 100/1, preferably from 30/1 to 90/1. The reaction product leaving the alkylation guard bed essentially consists of 90-98% by eight of aromatic hydrocarbon and 2-10% by weight of alkylated product.

Analogously, a fraction A2, equal to 20-40% of the overall aromatic stream is fed to the section of the guard reactor containing the transalkylation catalytic bed, together with a third stream essentially consisting of dialkylated aromatic hydrocarbons, of step (c) with A2/olefin molar ratios, calculated with respect to the total moles of olefin groups present as substituents in the dialkylated aromatic hydrocarbons, ranging from 2/1 to 18/1, preferably from 2.5/1 to 10/1. The reaction product leaving the transalkylation guard bed essentially consists of 80-90% by weight of aromatic hydrocarbon and 2-10% by weight of mono-alkylated product, the complement to 100 essentially consisting of dialkylated product.

The respective reaction products leaving the two guard beds in turn form the feedstocks to the alkylation and transalkylation reactors of the alkylation section (a).

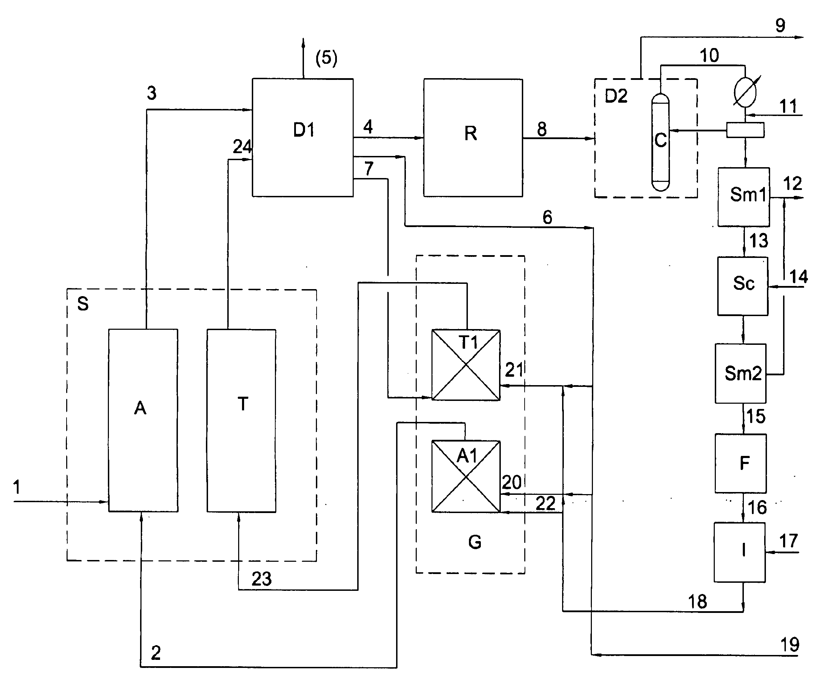

The integrated process for the production of vinyl aromatic hydrocarbons, object of the present invention, can be better understood by referring to the scheme of the enclosed figure which represents an illustrative and nonlimiting embodiment.

With reference to the scheme, A and T respectively represent the alkylation unit and the transalkylation unit of the alkylation section S.

D1 represents a first separation section, R is a dehydrogenation unit, D2 represents a second separation section comprising a distillation column for the aromatic hydrocarbon C.

Sm1 is a first demixer, Sc is a washing tower or water/organic mixer, Sm2 is a second demixer, f a unit containing at least one percolation/filtration bed and I a hydrogenation unit.

G is a guard reactor inside which two separate catalytic beds are arranged, one for an alkylation reaction A1, the other for a transalkylation reaction T1.

The functioning of the process scheme illustrated in the enclosed figure is evident on the basis of what is described below.

The alkylation section S comprises an alkylation unit A and a transalkylation unit T. A C₂ or C₃ olefin stream (1), for example ethylene, is fed to the alkylation unit A, together with an aromatic hydrocarbon stream (2), for example benzene (fresh and recycled). A stream (23) consisting of aromatic hydrocarbon, for example fresh and recycled benzene, mixed with the dialkylated product, for example recycled diethyl benzene, is fed to the transalkylation unit.

The reaction products (3) and (24) are recovered from the alkylation unit A and from the transalkylation unit T and are fed to the first separation section D1, comprising a series of distillation columns. The mono-alkylated product, for example ethyl benzene, stream (4) is recovered from D1, together with the polyalkylated products, stream (5), which are sent for treatments, not illustrated in the figure, and also the non-reacted aromatic hydrocarbon (6) and the dialkylated product (7), recycled as described hereunder.

The mono-alkylated product (4) is fed to the dehydrogenation unit R from which the stream (8) is discharged, and fed to the second separation column D2 comprising the distillation column of the aromatic hydrocarbon, hereafter benzene, C. Among other products not illustrated, the vinyl aromatic monomer (9), for example styrene with a purity higher than 99.7% by weight is recovered from the section D2, and a stream containing benzene (10) from the head of the column C, equipped with a vapour condenser and collection tank for the reflux. A stream of polar solvent (11), for example demineralized water, is fed downstream of the vapour condenser of column C.

The product at the head of column C, after condensation, goes to the demixer Sm1 where the organic phase (benzene) is demixed from the polar solvent, for example demineralized water. The latter (12) is treated and discharged into the sewerage system.

According to a preferred embodiment of the process scheme, object of the present invention, the hydrocarbon stream (13) leaving the demixer Sm1, essentially consisting of benzene, can be further treated with demineralized water (14), for example in a washing tower Sc. The product leaving Sc goes to the second demixer Sm2 from which the benzene is recovered from the aqueous phase.

The benzene (15) then goes to the filtration/percolation section F comprising one or more beds of molecular sieves or acid earth.

According to a further embodiment of the present invention, the benzene (16) leaving the filtration/percolation unit can be sent to a hydrogenation unit I, together with a flow of hydrogen 17.

The purified benzene (18), the fresh feeding benzene (19), previously treated in a system not represented in this scheme, and the benzene (6) coming from the first separation section D1 are fed to the alkylation section S, by means of the guard reactor G.

More specifically, the stream of fresh feeding benzene (19) is joined to the stream of the (first) recycled benzene (6) and they are respectively fed together to the catalytic bed A1, by means of (20), together with a stream of ethylene (22), and to the catalytic bed T1, by means of (21), together with the dialkylated products (7) leaving the first separation section D1.

The streams leaving the catalytic bed A1 and the catalytic bed T1 respectively, of the guard reactor G, form the feedings (2) and (23) to the alkylation reactor A and the transalkylation reactor T of the alkylation section S.

The purified stream of the (second) recycled benzene (18) can be partially joined to the streams (20) and (21) or it can be totally joined to the stream (20) or stream (21).

An illustrative example is provided for a better understanding of the present invention and for its embodiment.

### EXAMPLE

A stream of 1,000 kg/h, consisting of 35% by weight of benzene and 60% of toluene and the remaining part of saturated and unsaturated paraffins is fed to the benzene column C, operating at a pressure at the head of 0.4 MPa and a temperature at the head of 130°C.

350 kg/h of demineralized water are also fed to the head of the column C under the temperature and pressure conditions existing in the column.

After demixing in Sm1, 350 kg/h of benzene are recovered, which is further treated with 350 kg/h of water in a washing tower.

At the end of the second treatment with water, 350 kg/h of benzene are obtained which are percolated through a bed of particles of montmorillonite having a volume of 3.5 m³ and maintained at a temperature of 40°C. The montmorillonite particles are substantially spherical with a diameter ranging from 0.25 to 1.25 mm.

After the purification phase, the benzene recovered is joined to 30,000 kg/h of a quota of a mixture of refinery benzene (purity 99.8%) and recycled benzene coming from the first separation D1 and is fed to the transalkylation bed T1 of a guard reactor together with 4,000 kg/h of diethyl benzenes. The transalkylation bed is a fixed bed containing Y zeolite as catalyst and operating at T = 200°C and P = 3 MPa.

The remaining quota of a mixture of refinery benzene and recycled benzene from the first separation D1 equal to 80,000 kg/h, is fed to the alkylation bed A1 of the same guard reactor together with a stream of ethylene equal to 480 kg/h. The alkylation bed is a fixed bed containing beta zeolite as catalyst and operating at T = 220°C and P = 4 MPa.

The outlet of the transalkylation bed and alkylation bed are fed to a transalkylation reactor T and an alkylation reactor A respectively. The temperature and pressure operating conditions of the transalkylation and alkylation reactors are the same as those indicated for the respective guard reactor beds and the same with respect to the type of reactor and catalyst used. A stream of ethylene equal to 5,300 kg/h is also fed to the alkylation reactor.

The effluents of the alkylation and transalkylation reactors are then sent to the conventional separation section D1 for the recovery of the products, the non-reacted benzene, recycled, and by-products. The catalytic volume of the alkylation bed in the guard reactor is 20% of the total catalytic volume of the alkylation catalyst. The catalytic volume of the transalkylation bed in the guard reactor is 30% of the total catalytic volume of the transalkylation catalyst.

With this plant configuration, after 4000 h of functioning, the catalyst contained in the alkylation reactor A and transalkylation reactor T showed a loss in catalytic activity equal to 8% (measured as a variation in the thermal profile for the alkylation reaction and as a variation in the conversion of diethyl benzenes for the transalkylation reaction) whereas the loss in catalytic activity of the alkylation and transalkylation beds in the guard reactor proved to be equal to 16%.

The test was repeated, not effecting the washing of the benzene recovered from the head of the column C and using this recovered benzene, together with the mixture of refinery benzene and benzene recycled from the first separation, after treatment on a bed of montmorillonites particles under the same conditions described above.

With the same total catalytic volumes, after 4,000 h of functioning the catalyst contained in the alkylation and transalkylation reactors showed a loss in catalytic activity equal to 25% (measured on the basis of the variation in the thermal profile for the alkylation reaction and as a variation in the conversion of diethyl benzenes for the transalkylation reaction).

A complete loss of activity in the first test was therefore verified after 4,000 h on a quota of catalyst estimated at about 9.5% of the total against a complete loss in activity equal to 25% of the total in the second test.

For the second test, a total loss of catalytic activity of the alkylation and transalkylation reactor can therefore be estimated at 16,000 operating hours, whereas with the first test, the total loss of catalytic activity of the catalyst charged into the two beds of the guard reactor can be estimated at 24,000 hours with the catalyst charged into the main alkylation and transalkylation reactors still active for 50% of the volume charged. Consequently by only substituting the catalyst charged into the two beds of the guard reactor, it is possible to continue the run for a further 24,000 operating hours before reaching the complete loss in activity in the two main reactors.

## Claims

1. An integrated process for the production of vinyl aromatic hydrocarbons, which comprises:
a) feeding a hydrocarbon stream comprising a fresh and recycled aromatic hydrocarbon to an alkylation section, comprising a transalkylation unit, together with a stream essentially consisting of a C₂-C₃ olefin;
b) feeding the reaction product coming from the alkylation section to a first separation section;
c) discharging from the first separation section a first stream consisting of a non-reacted aromatic hydrocarbon which is recycled, a second stream essentially consisting of a mono-alkylated aromatic hydrocarbon, a third stream essentially consisting of dialkylated aromatic hydrocarbons and a fourth stream essentially consisting of a mixture of polyalkylated aromatic hydrocarbons;
d) feeding the second stream essentially consisting of the mono-alkylated aromatic hydrocarbon of step (c) to a dehydrogenation section to produce a vinyl aromatic hydrocarbon and reaction by-products comprising the aromatic hydrocarbon;
e) feeding the reaction product coming from the dehydrogenation step (d) to a second separation section, comprising a distillation column of the aromatic hydrocarbon;
f) recovering from said second separation section a stream consisting of the vinyl aromatic hydrocarbon, with a purity higher than 99.7% by weight, and a stream comprising the aromatic hydrocarbon;
g) recycling the aromatic hydrocarbon coming from the first and second separation section to the alkylation section comprising the transalkylation unit;
**characterized by**:
h) feeding a stream of polar solvent to the head of the distillation column of the aromatic hydrocarbon of step (e);
i) percolating the stream comprising the aromatic hydrocarbon coming from step (h) through at least one fixed bed consisting of molecular sieves or acid earth;
j) preparing a single guard reactor divided into two parts, the first containing a first catalytic bed, for an alkylation reaction, and the second containing a second catalytic bed, for a transalkylation reaction;
k) feeding to said first alkylation catalytic bed of said guard reactor, a first part (A1) of an overall aromatic stream comprising fresh feeding aromatic hydrocarbon, the recycled aromatic hydrocarbon coming from the first separation section (c) and from 0 to 100% by weight, preferably from 0 to 50% by weight, of the recycled aromatic hydrocarbon stream coming from the purification phase (h)-(i) together with a stream essentially consisting of a C₂-C₃ olefin, with A1/olefin molar ratios ranging from 10/1 to 100/1;
l) feeding to said second transalkylation catalytic bed of said guard reactor the second part (A2) of the overall aromatic stream comprising fresh feeding aromatic hydrocarbon, the recycled aromatic hydrocarbon coming from the first separation section (c) and from 0 to 100% by weight, preferably from 50 to 100% by weight, of the recycled aromatic hydrocarbon stream coming from the purification phase (h)-(i) together with the third stream, essentially consisting of dialkylated aromatic hydrocarbons, of step (c), with A2/olefin molar ratios, calculated with respect to the total moles of olefin groups present as substituents in the dialkylated aromatic hydrocarbons, ranging from 2/1 to 18/1; and
m) feeding the streams respectively leaving said first alkylation catalytic bed and said second transalkylation catalytic bed, of said single guard reactor, to the alkylation section comprising the transalkylation unit.

2. The process according to claim 1, wherein the aromatic hydrocarbon is benzene and the olefin is ethylene.

3. The process according to claim 1 or 2, wherein the polar solvent of step (h) is demineralized water.

4. The process according to any of the previous claims which comprises a second treatment of the aromatic hydrocarbon with the polar solvent in a second treatment unit.

5. The process according to any of the previous claims, wherein the molecular sieves or acid earth of step (i) are amorphous silico-aluminates with a silicon/aluminum ratio lower than 100.

6. The process according to claim 5, wherein the molecular sieve or acid earth is montmorillonite.

7. The process according to any of the previous claims, wherein the percolation of step (i) is effected at a temperature ranging from 25 to 300°C, at a pressure which is such as to maintain the aromatic hydrocarbon in the liquid state.

8. The process according to any of the previous claims which comprises treating the aromatic stream leaving the percolation in a hydrogenation unit operating at a pressure ranging from 0.5 to 2 MPa, at a temperature ranging from 80 to 150°C and in the presence of palladium supported on alumina.

9. The process according to any of the previous claims, wherein the parts of the guard reactor operate under the same conditions as the main reactors of the alkylation section.

10. The process according to any of the previous claims, wherein the fraction A1 of step (k) is equal to 60-80% of the overall aromatic stream.

11. The process according to any of the previous claims, wherein the fraction A2 of step (1) is equal to 20-40% of the overall aromatic stream.

12. The process according to any of the previous claims, wherein the A1/olefin and A2/olefin ratios respectively range from 30/1 to 90/1 and from 2.5/1 to 10/1.

## Patentansprüche

1. Integriertes Verfahren für die Herstellung von vinylaromatischen Kohlenwasserstoffen, welches umfasst:
a) Zuführen eines Kohlenwasserstoffstroms umfassend frischen und rezyklierten aromatischen Kohlenwasserstoff an eine Alkylierungssektion, umfassend eine Transalkylierungseinheit, zusammen mit einem Strom der im wesentlichen aus einem C₂-C₃ Olefin besteht;
b) Zuführen des Reaktionsprodukts das aus der Alkylierungssektion kommt zu einer ersten Trennungssektion;
c) Entnahme aus der ersten Trennungssektion eines ersten Stroms bestehend aus einem nicht umgesetzten aromatischen Kohlenwasserstoff welcher rezykliert wird, eines zweiten Stroms im wesentlichen bestehend aus monoalkyliertem aromatischen Kohlenwasserstoff, eines dritten Stroms im wesentlichen bestehend aus dialkylierten aromatischen Kohlenwasserstoffen, und eines vierten Stroms im wesentlichen bestehend aus einer Mischung aus polyalkylierten aromatischen Kohlenwasserstoffen;
d) Zuführen des zweiten Stroms im wesentlichen bestehend aus monoalkyliertem aromatischen Kohlenwasserstoff des Schritts (c) an eine Dehydrierungssektion um einen vinylaromatischen Kohlenwasserstoff und Reaktionsnebenprodukte umfassend den aromatischen Kohlenwasserstoff zu erzeugen;
e) Zuführen des Reaktionsprodukts aus dem Dehydrierungsschritt (d) zu einer zweiten Trennungssektion umfassend eine Destillationskolonne für den aromatischen Kohlenwasserstoff;
f) Gewinnen aus der zweiten Trennungssektion eines Stroms bestehend aus dem vinylaromatischen Kohlenwasserstoff mit einer Reinheit von mehr als 99,7Gew.-%, sowie eines Stroms umfassend den aromatischen Kohlenwasserstoff;
g) Rezyklieren des aromatischen Kohlenwasserstoffs der aus der ersten und zweiten Trennungssektion kommt zur Alkylierungssektion umfassend die Transalkylierungseinheit;
**gekennzeichnet durch**:
h) Zuführen eines Stroms von polarem Lösemittel zum Kopf der Destillationskolonne für den aromatischen Kohlenwasserstoff des Schritts (e);
i) Perkolieren des Stroms umfassend den aromatischen Kohlenwasserstoff kommend aus Schritt (h) **durch** mindestens ein Festbett bestehend aus Molekularsieben oder sauren Erden;
j) Bereiten eines einzelnen Schutzreaktors aufgeteilt in zwei Teile, der Erste enthaltend ein erstes katalytisches Bett für eine Alkylierungsreaktion, und der Zweite enthaltend ein zweites katalytisches Bett für eine Transalkylierungsreaktion;
k) Zuführen zu dem ersten Alkylierungskatalysebett des Schutzreaktors eines ersten Teils (A1) eines gesamtaromatischen Stroms umfassend frischen aromatischen Ausgangskohlenwasserstoff, des rezyklierten aromatischen Kohlenwasserstoffs kommend aus der ersten Trennungssektion (c) und 0 bis 100Gew.-%, vorzugsweise 0 bis 50Gew.-% des rezyklierten aromatischen Kohlenwasserstoffstroms kommend aus der Reinigungsphase (h) bis (i) zusammen mit einem Strom im wesentlichen bestehend aus einem C₁ bis C₃ Olefin, mit A1/Olefin Mol-Verhältnissen im Bereich von 10/1 bis 100/1;
l) Zuführen zu dem zweiten Transalkylierungskatalysebett des Schutzreaktors des zweiten Teils A2 des gesamten aromatischen Stroms umfassend frischen aromatischen Ausgangskohlenwasserstoff, des rezyklierten aromatischen Kohlenwasserstoffs kommend aus der ersten Trennungssektion (c) und von 0 bis 100Gew.-%, vorzugsweise von 50 bis 100Gew.-% des rezyklierten aromatischen Kohlenwasserstoffstroms kommend aus der Reinigungsphase (h) bis (i) zusammen mit dem drittem Strom, im wesentlichen bestehend aus dialkylierten aromatischen Kohlenwasserstoffen aus Schritt (c), mit A2/Olefin Mol-Verhältnissen im Bereich von 2:1 bis 18:1, berechnet bezüglich der gesamten Mole der Olefingruppen die als Substituenten in den dialkylierten aromatischen Kohlenwasserstoffen vorliegen; und
m) Zuführen des Stroms welcher das erste Alkylierungskatalysebett und das zweite Transalkylierungskatalysebett des genannten einzelnen Schutzreaktors verlässt zur Alkylierungssektion umfassend die Transalkylierungseinheit.

2. Verfahren nach Anspruch 1, wobei der aromatische Kohlenwasserstoff Benzol und das Olefin Ethylen ist.

3. Verfahren nach Anspruch 1 oder 2, wobei das polare Lösungsmittel des Schritts (h) entmineralisiertes Wasser ist.

4. Verfahren nach einem der vorhergehenden Ansprüche, welches eine zweite Behandlung des aromatischen Kohlenwasserstoffs mit dem polaren Lösemittel in einer zweiten Behandlungseinheit umfasst.

5. Verfahren nach einem der vorhergehenden Ansprüche, wobei die Molekularsiebe oder sauren Erden des Schritts (i) amorphe Silikoaluminate mit einem Silizium/Aluminiumverhältnis von weniger als 100 sind.

6. Verfahren nach Anspruch 5, wobei das Molekularsieb oder die saure Erde Montmorillonit ist.

7. Verfahren nach einem der vorhergehenden Ansprüche, wobei die Perkolation des Schritts (i) bei einer Temperatur im Bereich von 25 bis 300°C erfolgt, bei einem Druck der den aromatischen Kohlenwasserstoff im flüssigen Zustand hält.

8. Verfahren nach einem der vorhergehenden Ansprüche umfassend das Behandeln des aromatischen Stroms der die Perkolation verlässt in einer Hydriereinheit die bei einem Druck im Bereich von 0,5 bis 2 MPa betrieben wird bei einer Temperatur im Bereich von 80 bis 150°C und in Gegenwart von Palladium geträgert auf Aluminiumoxid.

9. Verfahren nach einem der vorhergehenden Ansprüche, wobei die Teile des Schutzreaktors unter den gleichen Bedingungen wie im Hauptreaktor für die Alkylierungssektion betrieben werden.

10. Verfahren nach einem der vorhergehenden Ansprüche, wobei die Fraktion A1 des Schritts (k) gleich 60 bis 80% des gesamten Aromatenstroms ausmacht.

11. Verfahren nach einem der vorhergehenden Ansprüche, wobei die Fraktion A2 des Schritts (1) 20 bis 40% des gesamten Aromatenstroms ausmacht.

12. Verfahren nach einem der vorhergehenden Ansprüche, wobei die A1/Olefin und A2/Olefin Verhältnisse im Bereich von 30/1 bis 90/1 bzw. 2,5/1 bis 10/1 liegen.

## Revendications

1. Procédé intégré pour la production d'hydrocarbures vinylaromatiques, qui comprend :
a) l'introduction d'un courant d'hydrocarbures comprenant des hydrocarbures aromatiques frais et recyclés dans une section d'alkylation, comprenant une unité de trans-alkylation, conjointement avec un courant constitué essentiellement d'une oléfine en C₂ à C₃ ;
b) l'introduction du produit réactionnel provenant de la section d'alkylation dans une première section de séparation ;
c) la décharge, à partir de la première section de séparation, d'un premier courant constitué d'un hydrocarbure aromatique n'ayant pas réagi qui est recyclé, d'un deuxième courant constitué essentiellement d'un hydrocarbure aromatique monoalkylé, d'un troisième courant constitué essentiellement d'hydrocarbures aromatiques dialkylés, et d'un quatrième courant constitué essentiellement d'un mélange d'hydrocarbures aromatiques polyalkylés ;
d) l'introduction du deuxième courant, constitué essentiellement de l'hydrocarbure aromatique monoalkylé de l'étape (c), dans une section de déshydrogénation pour produire un hydrocarbure vinylaromatique et des sous-produits réactionnels comprenant l'hydrocarbure aromatique ;
e) l'introduction du produit réactionnel provenant de l'étape de déshydrogénation (d) dans une deuxième section de séparation, comprenant une colonne de distillation de l'hydrocarbure aromatique ;
f) la récupération, à partir de ladite deuxième section de séparation, d'un courant constitué de l'hydrocarbure vinylaromatique, présentant une pureté supérieure à 99,7% en poids, et d'un courant comprenant l'hydrocarbure aromatique ;
g) le recyclage de l'hydrocarbure aromatique provenant des première et deuxième sections de séparation vers la section d'alkylation comprenant l'unité de trans-alkylation ;
**caractérisé par** :
h) l'introduction d'un courant de solvant polaire en tête de la colonne de distillation de l'hydrocarbure aromatique de l'étape (e) ;
i) la percolation du courant comprenant l'hydrocarbure aromatique provenant de l'étape (h) à travers au moins un lit fixe constitué de tamis moléculaire ou de terre acide ;
j) la préparation d'un seul réacteur de garde divisé en deux parties, la première contenant un premier lit catalytique, pour une réaction d'alkylation, et la deuxième contenant un deuxième lit catalytique, pour une réaction de trans-alkylation ;
k) l'introduction, dans ledit premier lit catalytique d'alkylation dudit réacteur de garde, d'une première partie (A1) d'un courant globalement aromatique comprenant une charge fraîche d'hydrocarbure aromatique, l'hydrocarbure aromatique recyclé provenant de la première section de séparation (c) et de 0 à 100 % en poids, de préférence de 0 à 50 % en poids, du courant d'hydrocarbures aromatiques recyclés provenant de la phase de purification (h) - (i) conjointement avec un courant essentiellement constitué d'une oléfine en C₂ à C₃, les rapports molaires A1/oléfine étant situés dans la plage allant de 10/1 à 100/1 ;
l) l'introduction, dans ledit deuxième lit catalytique de trans-alkylation dudit réacteur de garde, de la deuxième partie (A2) du courant globalement aromatique comprenant une charge fraîche d'hydrocarbure aromatique, l'hydrocarbure aromatique recyclé provenant de la première section de séparation (c) et de 0 à 100 % en poids, de préférence de 50 à 100 % en poids, du courant d'hydrocarbures aromatiques recyclés provenant de la phase de purification (h) - (i) conjointement avec le troisième courant, constitué essentiellement d'hydrocarbures aromatiques dialkylés, de l'étape (c), les rapports molaires A2/oléfine, calculés par rapport au total des moles des groupes oléfine présents en tant que substituants dans les hydrocarbures aromatiques dialkylés, étant situés dans la plage allant de 2/1 à 18/1 ; et
m) l'introduction des courants quittant respectivement ledit premier lit catalytique d'alkylation et ledit deuxième lit catalytique de trans-alkylation, dudit seul réacteur de garde, dans la section d'alkylation comprenant l'unité de trans-alkylation.

2. Procédé selon la revendication 1, dans lequel l'hydrocarbure aromatique est le benzène et l'oléfine est l'éthylène.

3. Procédé selon la revendication 1 ou 2, dans lequel le solvant polaire de l'étape (h) est l'eau déminéralisée.

4. Procédé selon l'une quelconque des revendications précédentes, qui comprend un deuxième traitement de l'hydrocarbure aromatique avec le solvant polaire dans une deuxième unité de traitement.

5. Procédé selon l'une quelconque des revendications précédentes, dans lequel le tamis moléculaire ou la terre acide de l'étape (i) est constitué d'aluminosilicates amorphes présentant un rapport silicium/ aluminium inférieur à 100.

6. Procédé selon la revendication 5, dans lequel le tamis moléculaire ou la terre acide est la montmorillonite.

7. Procédé selon l'une quelconque des revendications précédentes, dans lequel la percolation de l'étape (i) est effectuée à une température située dans la plage allant de 25 à 300°C, sous une pression telle que l'hydrocarbure aromatique soit maintenu à l'état liquide.

8. Procédé selon l'une quelconque des revendications précédentes, qui comprend le traitement du courant aromatique quittant la percolation dans une unité d'hydrogénation opérant sous une pression située dans la plage allant de 0,5 à 2 MPa, à une température située dans la plage allant de 80 à 150°C et en présence de palladium supporté sur de l'alumine.

9. Procédé selon l'une quelconque des revendications précédentes, dans lequel les parties du réacteur de garde opèrent dans les mêmes conditions que les réacteurs principaux de la section d'alkylation.

10. Procédé selon l'une quelconque des revendications précédentes, dans lequel la fraction A1 de l'étape (k) est égale à 60 à 80 % du courant total d'aromatiques.

11. Procédé selon l'une quelconque des revendications précédentes, dans lequel la fraction A2 de l'étape (k) est égale à 20 à 40 % du courant total d'aromatiques.

12. Procédé selon l'une quelconque des revendications précédentes, dans lequel les rapports A1/ oléfine et A2/oléfine sont respectivement situés dans les plages allant de 30/1 à 90/1 et de 2,5/1 à 10/1.
